# EUROPEAN PATENT SPECIFICATION

(11) **EP 2 620 498 B1**
(45) Date of publication and mention of the grant of the patent: **25.10.2017**
(21) Application number: 11826840.8
(22) Date of filing: 20.09.2011
(51) Int. Cl.: C12N 15/10, C12Q 1/68

(54) **METHOD FOR ACQUISITION OF SMALL RNA**
VERFAHREN ZUR ERFASSUNG KLEINER RNA
PROCÉDÉ POUR L'ACQUISITION DE PETIT ARN

(30) Priority: 24.09.2010 JP 2010214309
(43) Date of publication of application: 31.07.2013
(73) Proprietor: Wako Pure Chemical Industries, Ltd., Osaka-shi Osaka 540-8605 (JP)
(72) Inventor: NISHIBU Takahiro, Amagasaki-shi Hyogo 661-0963 (JP); HIRAYASU Kazunari, Amagasaki-shi Hyogo 661-0963 (JP)
(74) Representative: dompatent von Kreisler Selting Werner - Partnerschaft von Patent- und Rechtsanwälten mbB
(86) International application number: PCT/JP2011/071379
(87) International publication number: WO 2012/039395

(56) References cited:
- JP-A- 2009 148 243
- HAYASHIDA YUKINOBU ET AL: "A useful approach to total analysis of RISC-associated RNA", BMC RESEARCH NOTES, BIOMED CENTRAL LTD, GB, vol. 2, no. 1, 26 August 2009 (2009-08-26) , page 169, XP021060755, ISSN: 1756-0500, DOI: 10.1186/1756-0500-2-169
- M. JUNG ET AL: "Robust MicroRNA Stability in Degraded RNA Preparations from Human Tissue and Cell Samples", CLINICAL CHEMISTRY, vol. 56, no. 6, 8 April 2010 (2010-04-08), pages 998-1006, XP055096334, ISSN: 0009-9147, DOI: 10.1373/clinchem.2009.141580
- TAKAHIRO NISHIBU ET AL.: 'Ko Argonaute Kotai o Mochiita microRNA Kenkyu Tool no Shokai' WAKO JUN'YAKU JIHO vol. 78, no. 2, 15 April 2010, pages 8 - 11
- TAKAHIRO NISHIBU ET AL.: 'Kotai o Mochiita microRNA no Kino Kaisekiho -Atarashii microRNA Kenkyu Shiyaku no Katsuyo' BIO CLINICA vol. 24, no. 8, 2009, pages 58 - 62
- YUKINOBU HAYASHIDA ET AL.: 'microRNA Seisei to Cloning no Shin Shuho' MEDICAL BIO vol. 5, no. 2, 2008, pages 64 - 66

## Description

### Technical Field

The present invention relates to a method for acquisition of small RNA suitable for high throughput.

### Background Art

As the method for acquisition of small RNA such as microRNA (miRNA) and piwi-interacting RNA (piRNA) from biological sample such as a cell, the method in which, by immunoprecipitation reaction using a carrier bound, for example, with anti-Argonaute family antibody such as anti-Ago2 antibody, an Argonaute family protein-small RNA complex is obtained, and by separating small RNA from this complex, the small RNA is obtained, has been known (Patent reference 1). In the said method, as a method for separating small RNA from the Argonaute family protein-small RNA complex bound with the carrier, a method for extracting small RNA with the use of a phenol/chloroform/isoamyl alcohol mixed solution or an acidic guanidine/phenol/chloroform mixed solution is generally employed.

[Patent reference 1]: JP-A-2009-148243

### Disclosure of the Invention

### Problem to be solved by the Invention

In the separation method employing above-described extraction procedure, an operation of separating aqueous phase and organic phase by centrifugation was needed, and further, precipitation and concentration of the small RNA from the aqueous phase separated using alcohol such as ethanol and isopropanol had to be carried out. Moreover, since protein denaturing agents such as strong acidic buffer solution and surface-active agent usually used in elution of antigen from the antibody-antigen complex bound to the carrier by immunoprecipitation may inhibit enzyme reaction, the solution after elution is not able to use directly for the reaction system such as reverse transcription reaction and PCR. Hence, there was a problem that such method cannot be applied for the methods such as high throughput which have to be carried out by various types of processes continuously. Therefore, the development of a method which can be applied for high throughput and enables to obtain the small RNA simply and efficiently has been desired.

The present inventors have found, as a result of extensive investigation in view of above-described situation, that, in the immunoprecipitation method, the small RNA could be eluted from the complex by heating the carrier bound with Argonaute family protein-small RNA complex in a specified solution, and the eluate can be used directly for the subsequent reaction system, and thus completed the present invention. That is, the purpose of the present invention is to provide a method which can be applied for high throughput and enables to obtain the small RNA simply and efficiently.

### Means for solving the Problems

The present invention relates to "a method for acquisition of small RNA having 5 to 200 nt, said method comprising contacting a carrier, which comprises a substance having affinity to a small RNA-binding Argonaute-family protein immobilized on its surface, with a complex of the small RNA-binding Argonaute-family protein and a small RNA (Argonaute-family protein-RNA complex) to bind the Argonaute-family protein-RNA complex to the aforementioned carrier, and releasing the small RNA by heating the carrier bound with the aforementioned Argonaute-family protein-RNA complex at 70°C to 100°C for 30 to 600 s in water or buffer solution of pH 3.0 to pH 8.0".

### Effect of the Invention

Since, in the method of the present invention, there is no necessity of conducting extraction and precipitation procedures nor necessity of using protein denaturation agents such as surface-active agent, as compared with the conventional method, the eluate obtained by the method of the present invention can be used directly for the reaction such as reverse transcription reaction and PCR, and thus, the method of the present invention can be incorporated as one process in a screening by the high throughput method. Therefore, the method of the present invention enables to acquire small RNA simply. Particularly, when a magnetic material is used as a carrier, even if the particle sizes of the carrier is small, there is no necessity of performing centrifugation after immunoprecipitation reaction, and small RNA can be obtained more simply and easily.

Furthermore, in the method of the present invention, also with respect to acquisition efficiency of small RNA, the small RNA can be obtained at efficiency of equivalent to or higher than the conventional method. Therefore, according to the method of the present invention, even when the number of samples is large, acquisition of small RNA is enabled to perform in a short period of time. Hence the present method is suitable for research and diagnosis where a large number of samples have to be processed.

### Brief Description of the Drawings

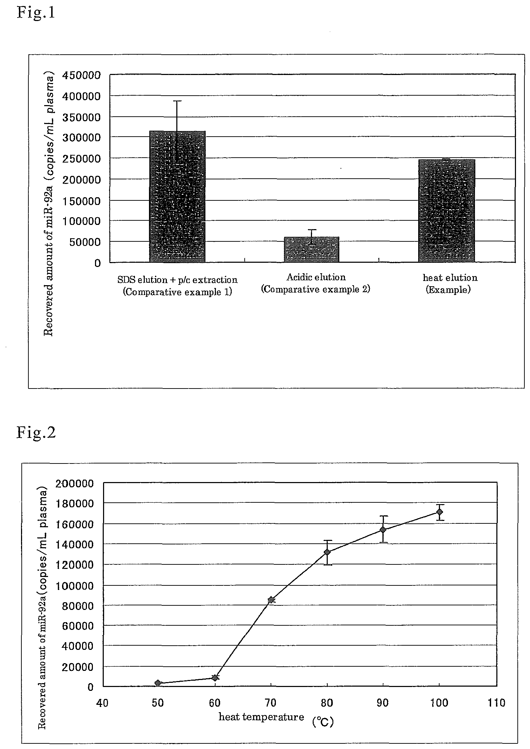

Fig. 1 shows the results obtained in Example 1, Comparative Example 1 and Comparative Example 2, showing the amount of miRNA (miR-92a) recovered using various extraction methods after human plasma is immunoprecipitated with anti-human Ago2 antibody-immobilized beads. Starting from the left, the results recovered by SDS elution-phenol/chloroform extraction, the results recovered by acidic elution method, the results recovered by the heat elution method of the present invention, are shown, respectively.
Fig. 2 shows the results obtained in Example 2, showing the amount of miRNA (miR-92a) obtained by heat elution at 50°C, 60°C, 70°C, 80°C, 90°C, 100°C, after human plasma is immunoprecipitated with anti-human Ago2 antibody-immobilized beads, and then added with nuclease-free water.

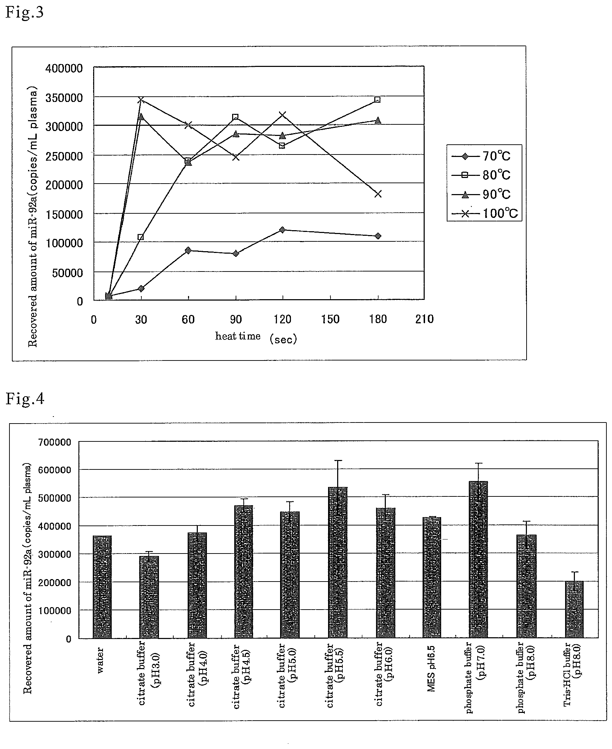

Fig. 3 shows the results obtained in Example 3, showing the amount of miRNA (miR-92a) obtained by heat elution at 70°C, 80°C, 90°C, 100°C for 10 s, 30 s, 60 s, 90 s, 120 s, and 180 s for respective temperature after human plasma is immunoprecipitated with anti-human Ago2 antibody-immobilized beads, and then added with nuclease-free water.
Fig. 4 shows the results obtained in Example 4, showing the amount of miRNA (miR-92a) obtained by heat elution at 90°C for 90 s after human plasma is immunoprecipitated with anti-human Ago2 antibody-immobilized beads, and then added with various buffer solution. Starting from the left, the results obtained by using nuclease-free water, 10 mM citrate buffer solution (pH 3.0), 10 mM citrate buffer solution (pH 4.0), 10 mM citrate buffer solution (pH 4.5), 10 mM citrate buffer solution (pH 5.0), 10 mM citrate buffer solution (pH 5.5), 10 mM citrate buffer solution (pH 6.0), 10 mM MES buffer solution (pH 6.5), 10 mM phosphate buffer solution (pH 7.0), 10 mM phosphate buffer solution (pH 8.0), 10 mM Tris-HCl buffer solution (pH 8.0) as the various buffer solutions, are shown.

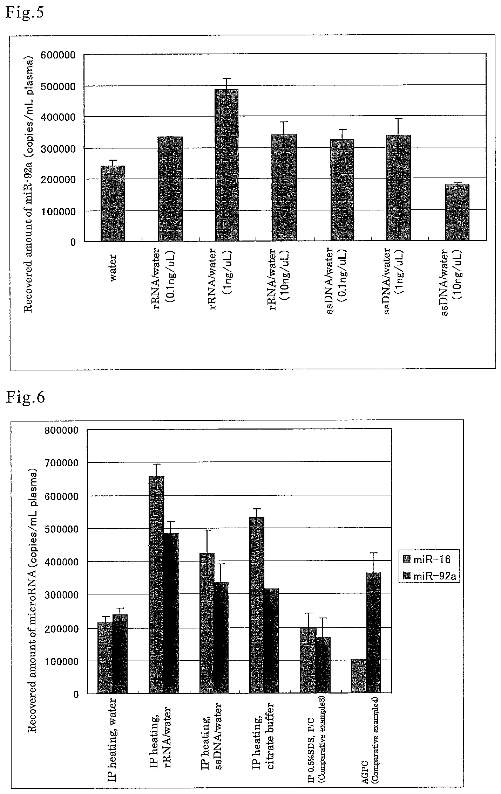

Fig. 5 shows the results obtained in Example 5, showing the amount of miRNA (miR-92a) obtained by heat elution at 90°C for 90 s after human plasma is immunoprecipitated with anti-human Ago2 antibody-immobilized beads, and then added with nuclease-free water with or without various nucleic acids. Starting from the left, the result obtained by using additive-free nuclease-free water, the results obtained by using nuclease-free water added with each 0.1 ng/µl, 1 ng/µl, and 10 ng/µl final concentration of E. coli ribosomal RNA, and the results obtained by using nuclease-free water added with each 0.1 ng/µl, 1 ng/µl, and 10 ng/µl final concentration of salmon sperm DNA as the nuclease-free water added with various nucleic acids, are shown.
Fig. 6 shows the results obtained in Example 6, Comparative Example 3 and Comparative Example 4, showing the amount of miR-16 and miR-92a recovered from human plasma by various methods. Starting from the left, the amount of miRNA obtained when heat elution is carried out with nuclease-free water after immunoprecipitation with anti-human Ago2 antibody-immobilized beads; the amount of miRNA obtained when heat elution is carried out with nuclease-free water added with E. coli ribosomal RNA (final concentration: 1 ng/µl) after immunoprecipitation with anti-human Ago2 antibody-immobilized beads; the amount of miRNA obtained when heat elution is carried out with nuclease-free water added with salmon sperm DNA (final concentration: 1 ng/µl) after immunoprecipitation with anti-human Ago2 antibody-immobilized beads; the amount of miRNA obtained when heat elution is carried out with 10 mM citrate buffer solution (pH 5.5) after immunoprecipitation with anti-human Ago2 antibody-immobilized beads; the amount of miRNA obtained when SDS elution- phenol/chloroform extraction is carried out after immunoprecipitation with anti-human Ago2 antibody-immobilized beads; and the amount of miRNA obtained when human plasma is extracted with AGPC; are shown respectively.

### Best Mode for Carrying Out the Invention

The small RNA pertaining to the present invention may be RNA comprising usually 5 to 200 nt, preferably 10 to 50 nt, more preferably 10 to 30 nt, and is preferably the one in which a protein to be bound thereto is present. The small RNA pertaining to the present invention include 5S ribosomal RNA (5SrRNA), transfer RNA (tRNA), small nuclear RNA (snRNA), small nucleolar RNA (snoRNA), transcription initiation RNA (tiRNA), splice site RNA (spliRNA), small interfering RNA (siRNA), piwi-interacting RNA (piRNA) and micro RNA (miRNA); among them, piRNA, siRNA and miRNA, which are known to bind to Argonaute-family protein, are preferable, and miRNA is particularly preferable.

The small RNA-binding protein pertaining to the present invention may vary depending on the type of target small RNA, and is an Argonaute-family protein which is capable of binding with small RNA pertaining to the above-described present invention. Said Argonaute-family protein includes specifically Argonaute subfamily such as Ago1, Ago2, Ago3, and Ago4, and Piwi subfamily such as PiwiL1, PiwiL2, PiwiL3, and PiwiL4, among them, the Argonaute subfamily is preferable, and Ago2 is particularly preferable.

A complex of small RNA-binding Argonaute-family protein and small RNA pertaining to the present invention (hereinafter, sometimes, abbreviated to Argonaute-family protein-RNA complex pertaining to the present invention) is a complex formed by binding small RNA-binding Argonaute-family protein pertaining to the present invention and small RNA pertaining to the present invention. The Argonaute-family protein-RNA complex pertaining to the present invention also includes the ones which further comprise protein to be bound to said complex (for example, TNRC6A, Gemin3, Gemin4 and FMRP). As a specific example of said complex, for example, RISC (RNA-induced silencing complex) are included.

The substance which has affinity to the small RNA-binding Argonaute-family protein (hereinafter, optionally abbreviated as an Argonaute-family protein affinity substance pertaining to the present invention) may be the one which is capable of binding to a substance having affinity to the small RNA-binding Argonaute-family protein, and depending on the type of the above-described small RNA-binding Argonaute-family protein. It includes antibody which has affinity (binding ability) to the small RNA-binding Argonaute-family protein, and aptamer. Among them, antibody is preferable. Specifically, for example, when Ago2 is employed as a small RNA-binding Argonaute-family protein, anti-Ago2 antibody is used. When Ago3 is employed, anti-Ago3 antibody is used.

The origin of the antibody to be used as an Argonaute-family protein affinity substance pertaining to the present invention is not particularly limited, and may be either a polyclonal antibody or a monoclonal antibody. Monoclonal antibody is more preferable. In addition, as for these antibodies, commercially available antibody products may be employed, and if needed, F(ab')₂, Fab' or Fab, which is prepared by digesting using an enzyme such as pepsin and papain may be used.

As the method for obtaining the above-described polyclonal antibody, the polyclonal antibody may be prepared according to the method described in "Nao Matsuhashi, et al., Introduction of Experimental Immunology, 2nd printing, Japan Scientific Society Press, 1981" by the common procedure for immunizing an animal such as, for example, horse, cattle, sheep, rabbit, goat, guinea pigs, rat, and mouse with an objective substance to be measured. In addition, the method for obtaining monoclonal antibody includes a method in which an immunologically sensitized cell such as, for example, spleen cell, lymphocyte of an animal such as, for example, rat, mouse which has been immunized against the objective substance to be measured as an immunogen, and a cell which has a property of immortal growth, such as, for example, myeloma cell are fused to produce a hybridoma by the cell fusion technology well known per se developed by Kohler and Milstein (Nature, 256, 495, 1975), then said hybridoma is cultured in a culture medium or injected into the intraperitoneal of animals to produce the antibody in ascites, and the objective monoclonal antibody is harvested from said culture medium or ascites, and a method in which the antibody-producing cells having the property as described above are produced by a method (Eur. J. Immunol., 6, 511, 1976) well known per se utilizing, for example, recombinant DNA technologies, and by culturing these cells to obtain the objective monoclonal antibody.

In addition, as the method for preparing the above-described aptamer, the method described in US patent No. 5,270,163 is preferable.

As the carrier pertaining to the present invention, on the surface of which a substance having affinity to small RNA-binding Argonaute-family protein is immobilized (hereinafter, optionally abbreviated as a carrier pertaining to the present invention), any carrier usually used in the immunological assay method can be employed. The carrier pertaining to the present invention includes, ones made of, for example, organic substances, such as polystyrene, polyacrylic acid, polymethacrylic acid, polymethyl methacrylate, polyacrylamide, polyglycidyl methacrylate, polypropylene, polyvinyl chloride, polyethylene, polychlorocarbonate, silicone resin, silicone rubber, agarose, dextran, and ethylene-maleic anhydride copolymer, inorganic substances such as porous glass, ground glass, alumina, silica gel, metal oxides, and magnetic substances such as iron, cobalt, nickel, magnetite, and chromite, as a material. In addition, these carriers can be used with various forms such as a tube, a bead, a disk-shaped piece, and a particle. Among them, usage in the form of particles is preferable, the size of particles is not particularly limited, but it is preferable to use the particle sizes of a few nm to 100 µm according to the purpose and application.

The carrier pertaining to the present invention also includes a carrier formed as a layer comprising high polymer compound on the surface of the carrier as described above, and such carrier is included as a preferable one because contaminating proteins are hardly adsorbed nonspecifically on the surface thereof, and addition of a blocking agent for preventing nonspecific adsorption is not required. The method of forming high polymer compound and a layer comprising said high polymer compound to be formed on the surface of the carrier may be carried out using polymer molecule described in JP-A-2009-148243 and according to the method described in the same. For example, to the above-described surface of the carrier pertaining to the present invention, a polymerizable functional group such as a vinyl group, an allyl group, a methacryl group, an epoxy group, and a styrene group, or a chain transfer group such as a mercapto group and an amino group is introduced; and the obtained carrier is mixed with ethylenically unsaturated polymerizable monomer having a functional group which immobilizes the Argonaute-family protein affinity substance pertaining to the present invention, and if necessary, an ethylenically unsaturated polymerizable monomer having an alkylene oxy group is further mixed; after that polymerization reaction is allowed to progress. Specifically, for example, it is obtained by the following procedure.

That is, for example, into an aqueous solution containing 0.01 mol/l to 1.0 mol/l of silane coupling agent such as methacryloxypropyltrimethoxysilane in acetic acid (for example, pH 2 to pH 4), 1 g to 10 g of silica beads are added, and reacted at 50°C to 100°C for 10 min to 180 min, to prepare the silica beads having a polymerizable functional group or a chain transfer group. On the other hand, p-nitrophenyl-oxycarbonyl poly(ethylene glycol) methacrylate and poly(ethylene glycol) methyl ether methacrylate are mixed, for example, so as to give a ratio of 1:99 to 70:30, and dissolved in a solvent such as dehydrated ethanol. Subsequently, after 1 to 10 times more mol of a polymerization initiator such as azodiisobutyronitrile (AIBN) is added to said solution and agitated, the silica beads are added so that 0.1 mmol to 10 mmol of the above-described polymer is present relative to 1 g of the silica beads having the above-described polymerizable functional group or the chain transfer group. The mixture is reacted at 50°C to 80°C for 10 h to 30 h, if necessary under argon atmosphere, followed by drying, and then the carrier, on the surface of which a layer comprising high polymer compound has been formed, is obtained.

The amount of the Argonaute-family protein affinity substance pertaining to the present invention which is to be immobilized on the carrier pertaining to the present invention may differ depending on the kind of Argonaute-family protein affinity substance to be used, however, usually, it is 0.1 mg to 10 mg for 1 g carrier, preferably 1 mg to 10 mg for 1 g carrier.

The carrier pertaining to the present invention, on the surface of which a substance having affinity to small RNA-binding Argonaute-family protein has been immobilized (hereinafter, abbreviated to Argonaute-family protein affinity substance-immobilized carrier pertaining to the present invention), is the one in which the above-described Argonaute-family protein affinity substance pertaining to the present invention has been immobilized on the surface of the above-described carrier pertaining to the present invention.

The method for immobilizing the Argonaute-family protein affinity substance pertaining to the present invention on the surface of the carrier pertaining to the present invention includes immobilization method well known per se, for example, a method of immobilization by chemical bond such as covalent bond, a method of immobilization by physical adsorption (JP-5-41946), or immobilization method well known per se such as a method of immobilization by affinity binding through a substance having affinity for antibody such as protein A and protein G. Among them, the method of immobilization by chemical bond and the method of immobilization by affinity binding are preferable. In addition, when plasma or serum which contains large amount of antibody in its component is used as a sample (a solution containing the Argonaute-family protein-RNA complex pertaining to the present invention), the method of immobilization by chemical bond is preferable, especially the method in which the Argonaute-family protein affinity substance is immobilized using functional group introduced on the surface of the particle is particularly preferable. In this case, in accordance with the functional group in the Argonaute-family protein affinity substance, the functional group to be introduced may be selected appropriately. A specific method for binding the functional group introduced on the surface of particle with the functional group in the Argonaute-family protein affinity substance may be carried out as follows.

That is, when a carboxyl group is bound to a carboxyl group, binding may be performed using polyhydroxy compound or alkylene oxide as a condensation agent. 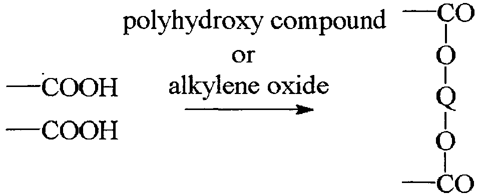 (wherein, -O-Q-O- represents a group derived from polyhydroxy compound or alkylene oxide.)

When a carboxyl group is bound to a carboxyl group, binding may be performed using polyamine compound as a condensation agent. 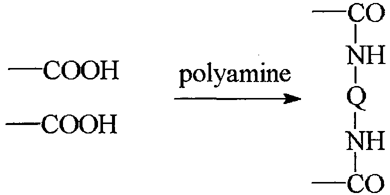 (wherein, -HN-Q-NH- represents a group derived from polyamine.)

When a hydroxyl group is bound to a hydroxyl group, binding may be performed using polycarboxylic acid as a condensation agent. 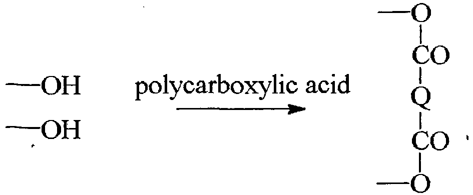 (wherein, -OOC-Q-COO- represents a group derived from polycarboxylic acid.)

When a hydroxyl group is bound to a hydroxyl group, binding may be performed using alkylene oxide as a condensation agent. 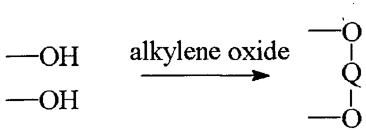 (wherein, -O-Q-O- represents a group derived from alkylene oxide.)

When an isocyanate group is bound to an isocyanate group, binding may be performed using water.

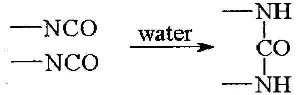

When an isocyanate group is bound to an isocyanate group, binding may be performed using polyhydroxy compound as a condensation agent. 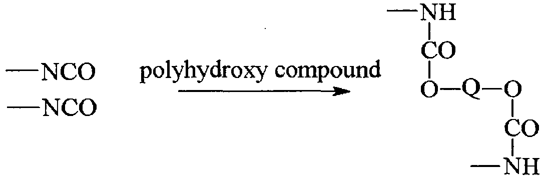 (wherein, -O-Q-O- represents a group derived from dihydroxy compound.)

When an isocyanate group is bound to an isocyanate group, binding may be performed using polycarboxylic acid as a condensation agent. 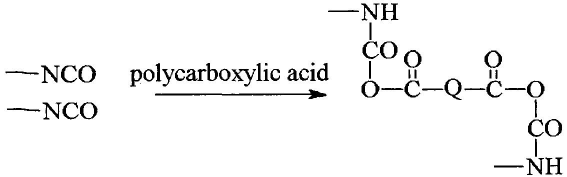 (wherein, -O-CO-Q-OC-O- represents a group derived from dicarbonic acid.)

When an isocyanate group is bound to an isocyanate group, binding may be performed using polyamine cross-linking agent. 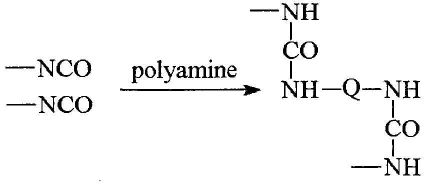 (wherein, -HN-Q-NH- represents a group derived from polyamine.)

When an amino group is bound to a carboxyl group, binding may be performed using dehydrating agent.

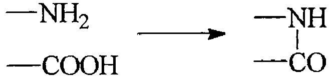

When a hydroxyl group is bound to carboxyl group, binding may be performed using dehydrating agent.

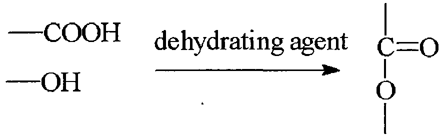

The method for immobilizing Argonaute-family protein affinity substance pertaining to the present invention on the surface of the carrier pertaining to the present invention, specifically, may be carried out, for example, by contacting 0.5 ml of a solution containing usually 2 µg/ml to 200 µg/ml, preferably 20 µg/ml to 200 µg/ml of Argonaute-family protein affinity substance pertaining to the present invention (a solution containing Argonaute-family protein affinity substance pertaining to the present invention) with 10 mg of the carrier pertaining to the present invention, if necessary under coexistence of appropriate condensation agent, and by reacting at usually 20°C to 50°C, preferably 30°C to 40°C for usually 1 h to 20 h, preferably 1 h to 10 h, more preferably 2 h to 5 h. Here, as the condensation agent to be used if needed, the one conventionally used in this field may be used appropriately, and the condensation agent described above in the section of combination of functional groups are included as a preferable one. In addition, after the Argonaute-family protein affinity substance is immobilized, it is desirable to perform a treatment with blocking agents such as ethanolamine, to inactivate the functional group on the surface of the carrier pertaining to the present invention to which the Argonaute-family protein affinity substance has not been immobilized.

The solvent to be used for preparing a solution of the Argonaute-family protein affinity substance pertaining to the present invention may be the one which does not have a property of preventing adsorption or binding of the Argonaute-family protein affinity substance to the insoluble carrier, for example, purified water, buffer solution (such as, for example, phosphate buffer solution, Tris buffer solution, Good's buffer solution, glycine buffer solution, borate buffer solution and sodium bicarbonate buffer solution) having a buffering action, for example, at pH 5.0 to pH 10.0, preferably pH 8.5 to pH 10 are preferable. In addition, concentration of the buffering agent in these buffer solutions is selected as appropriate from the range of usually 0.1 M to 5 M, and preferably 0.6 M to 2.5 M. In addition, in this solution, as long as the amount which does not interfere adsorption or binding of the said antibody to the insoluble carrier, for example, sugar, salts such as NaCl, surface-active agent, antiseptic agent and protein may be contained. In addition, the Argonaute-family protein affinity substance-immobilized carrier obtained as described above may be subjected to the blocking process usually performed in this field.

For example, when Ago2 is used as an Argonaute-family protein substance pertaining to the present invention, the Argonaute-family protein affinity substance-immobilized carrier pertaining to the present invention is prepared as follows: That is, at an existence ratio of 0.1 mg to 10 mg of the Argonaute-family protein affinity substance pertaining to the present invention such as anti-Ago2 antibody relative to 1 g of the carrier pertaining to the present invention, these are added to sodium bicarbonate buffer solution, and reacted at 30°C to 40°C for 2 h to 10 h. Thereby, the Argonaute-family protein affinity substance pertaining to the present invention is immobilized on the surface of the particle, and thus the Argonaute-family protein affinity substance-immobilized carrier is obtained.

The method for acquisition of small RNA of the present invention is performed by the following steps:
[Step (1)] Argonaute-family protein affinity substance-immobilized carrier pertaining to the present invention is contacted with the Argonaute-family protein-RNA complex pertaining to the present invention, and the Argonaute-family protein-RNA complex is bound to the relevant carrier;
[Step (2)] If needed, the carrier bound with the relevant Argonaute-family protein-RNA complex is washed;
[Step (3)] By heating the carrier bound with the relevant Argonaute-family protein-RNA complex in water or buffer solution of pH 3.0 to pH 8.0 at 70°C to 100°C for 30 to 600 s, the small RNA is released.

Specific method will be described below.

### The method for acquisition of the small RNA of the present invention [Step (1)]

Firstly, a solution containing Argonaute-family protein-RNA complex is mixed with the Argonaute-family protein affinity substance-immobilized carrier pertaining to the present invention, and reacted at 2°C to 37°C, preferably at 2°C to 10°C for 1 h to 30 h, preferably for 2 h to 10 h, more preferably for 2 h to 5 h to bind the Argonaute-family protein-RNA complex pertaining to the present invention to the Argonaute-family protein affinity substance-immobilized carrier pertaining to the present invention.

The solution containing the Argonaute-family protein-RNA complex to be used on this occasion includes, along with cell lysate (cell extract) containing Argonaute-family protein-RNA complex and conditioned cell culture medium, a body fluid such as plasma, serum, urine, saliva, and mother's milk, or a solution thereof dissolved or suspended in a solvent. In addition, for said cell lysate, usually, the one which 5 x 10⁶ to 1 x 10⁷ cells contained in 1 ml solution is lysed is used.

The solvent (reaction solvent) of the solution containing the Argonaute-family protein-RNA complex on the occasion of using cell lysate includes a buffer solution containing NaCl. For the purpose of acquisition of small RNA in more efficient, the NaCl-containing buffer solution which is further added with surface-active agent as a cell lysing agent usually used in this field is preferable. As the said buffer solution, a buffer solution having buffering action at near neutral, for example, at pH 5.0 to pH 10.0, preferably pH 6.5 to pH 8.5, for example, phosphate buffer solution, Tris buffer solution, Good's buffer solution, glycine buffer solution and borate buffer solution is preferable. In addition, concentration of the buffering agent in the buffer solution is selected appropriately from a range of usually 10 mM to 500 mM, preferably 10 mM to 300 mM. Concentration of NaCl is usually 100 mM to 200 mM as the concentration in the buffer solution. Further, the surface-active agent to be added includes, for example, poly(oxyethylene) nonylphenyl ether (produced by Wako Pure Chemical Industries Ltd.) and TritonX-100, however poly(oxyethylene) nonylphenyl ether is preferable. The concentration of the surface-active agent may be a concentration which does not affect the Argonaute-family protein affinity substance but has cell lysing effect, and which is usually 0.01% to 0.5%, preferably 0.05% to 0.1% to a total volume of the buffer solution.

In addition, in the case where conditioned cell culture medium or body fluid is used, the surface-active agent may be added as a lysing agent for cellular secretory granules (for example, exosome). The said surface-active agent may include, for example, poly(oxyethylene) nonylphenyl ether (produced by Wako Pure Chemical Industries Ltd.) and TritonX-100, however poly(oxyethylene) nonylphenyl ether is preferable. The additive concentration thereof may be the concentration which does not affect the Argonaute-family protein affinity substance but has cell lysing effect, and which is usually 0.01% to 0.5%, preferably 0.05% to 0.1% to total volume of the buffer solution.

In the above-described step (1), the amount of the Argonaute-family protein affinity substance-immobilized carrier pertaining to the present invention to be used is usually 1 mg to 10 mg, preferably 1 mg to 5 mg per 1 ml solution of the above-described Argonaute-family protein-RNA complex, and the amount of Argonaute-family protein affinity substance pertaining to the present invention to be immobilized on the carrier is usually 0.1 µg to 100 µg, preferably 1 µg to 50 µg.

### The method for acquisition of the small RNA of the present invention [Step (2)]

After the reaction of the above-described step (1), if needed, the carrier bound with Argonaute-family protein-RNA complex pertaining to the present invention is separated from reaction solution, and further, in order to remove the free Argonaute-family protein-RNA complex and cell-derived substances attached to the surface of the carrier, it is preferable to wash the obtained carrier with washing liquid.

The method for isolation of the carrier to which the above-described Argonaute-family protein-RNA complex pertaining to the present invention has been bound is done by removing the supernatant solution of said reaction solution, if needed, after subjecting the reaction solution of step (1) to centrifugal separation. Said centrifugal separation is not particularly limited so long as it is carried out in the manner usually carried out in this field, and may be performed by centrifugation, for example, at 1,000 x g to 10,000 x g for 10 s to 100 s. In addition, if the carrier is made of magnetic substance as a material, centrifugal separation is not necessary, and separation of carrier and supernatant solution can be performed using magnetic stand, and thereby the carrier can be isolated by removing the supernatant solution.

The washing liquid to be used in the above-described washing includes a buffer solution containing NaCl; however, the NaCl-containing buffer solution which is further added with surface-active agent as a cell lysing agent usually used in this field is preferable. As the said buffer solution, a buffer solution having buffering action at near neutral, for example, at pH 5.0 to pH 10.0, preferably pH 6.5 to pH 8.5, for example, phosphate buffer solution, Tris buffer solution, Good's buffer solution, glycine buffer solution and borate buffer solution is preferable. In addition, concentration of the buffering agent in the buffer solution is selected appropriately from a range of usually 10 mM to 500 mM, preferably 10 mM to 300 mM. Concentration of NaCl is usually 100 mM to 200 mM as the concentration in the buffer solution. Further, the surface-active agent to be added includes, for example, poly(oxyethylene) nonylphenyl ether (produced by Wako Pure Chemical Industries Ltd.) and TritonX-100, however poly(oxyethylene) nonylphenyl ether is preferable. The concentration of the surface-active agent may be a concentration which does not affect the Argonaute-family protein affinity substance but has cell lysing effect, and which is usually 0.01% to 0.5%, preferably 0.05% to 0.1% to a total volume of the buffer solution. The amount of washing liquid is 1 to 10 times of the amount of the reaction solution.

By carrying out washing procedure as described above, a contaminating substance such as biological substances adhering to the Argonaute-family protein affinity substance-immobilized carrier pertaining to the present invention can be removed; in particular, divalent cation such as Mg²⁺ and Ca²⁺ contained in the biological substances can be removed. Consequently, susceptibility to hydrolysis of small RNA by divalent cation under high temperature of 70°C to 100°C is avoided, and acquisition of small RNA in higher efficiency becomes possible. In addition, since the above-described washing procedure increases its effect by performing repeatedly, it is preferable to carry out repeatedly.

### The method for acquisition of the small RNA of the present invention [Step (3)]

Subsequently to step (1) or step (2), the carrier bound with Argonaute-family protein-RNA complex pertaining to the present invention is heated usually at 70°C to 100°C for 30 s to 600 s, in 10 µl to 100 µl, preferably 10 µl to 50 µl of water or buffer solution of pH 3.0 to pH 8.0 relative to 1 mg of the carrier. After that, if needed, after subjecting the relevant solution to centrifugal separation, and by performing preparative isolation of the supernatant solution of the said solution, a solution containing objective small RNA can be obtained. Such solution can be used directly as a sample for reverse transcription reaction and PCR reaction.

The water to be used in the above-described step (3) is not limited specifically as long as it is the one usually used in this field; however the one which is free of nucleases such as ribonuclease, deoxyribonuclease is preferable.

As the buffer solution to be used in the above-described step (3), since if it is strong alkaline, RNA would be hydrolyzed, its pH is in the range from 3.0 to 8.0, and the one having pH 4.5 to pH 7.0 at which recovery rate of RNA is high, and subsequent reactions are not inhibited is preferable. Type of buffer solution is not limited so long as it is within the above-described pH range, and specifically includes, for example, citrate buffer solution, Good's buffer solution such as MES (2-Morphorinoethanesulfonic acid), phosphate buffer solution and Tris buffer solution, among them, citrate buffer solution, Good's buffer solution and phosphate buffer solution are preferable, and citrate buffer solution and phosphate buffer solution are more preferable. It should be noted that, these buffer solutions may be used by mixing 2 or more types. The concentration of buffering agent in the said buffer solution is usually 5 mM to 100 mM, and 10 mM to 50 mM is preferable. By using these buffer solutions, it becomes possible to obtain small RNA with higher efficiency compared with that obtained using water.

Water or buffer solution to be used in the above-described step (3) may contain RNA which does not comprise nucleotide sequence of the target RNA, or DNA. The RNA to be dissolved in the water or the buffer solution includes, for example, rRNA (ribosomal RNA), tRNA (transfer RNA), and synthetic hairpin RNA, and among them, rRNA and synthetic hairpin RNA are preferable. The DNA includes, for example, the one derived from salmon sperm and one derived from λ-phage, and among them, the one derived from salmon sperm is preferable. The amount of RNA and DNA to be used and contained in the water or buffer solution may differ depending on the RNA or DNA used; however it is the amount that provides the concentration of usually 0.1 ng/µl to 10 ng/µl, preferably 0.1 ng/µl to 1 ng/µl in the solution. In addition, when RNA or DNA is dissolved in this way, the solution to be used is preferably water rather than buffer solution.

The heating temperature of the water or buffer solution used in the above-described step (3) is usually from 70°C to 100°C, but 80°C to 100°C is preferable, and 80°C to 90°C is more preferable. The heating time is set between 30 s and 600 s depending on the heating temperature, and specifically, for example at 70°C, it is from 120 s to 600 s; at 80°C, it is from 90 s to 600 s, at 90°C or higher, it is from 30 s to 600 s.

The centrifugal separation in the above-described step (3) is not specifically limited as long as it is an aspect usually carried out in this field, and it may be carried out at 1000 x g to 10000 x g for 10 s to 100 s.

In more specifically, for example, in the case where an anti-Ago2 antibody is used as an Argonaute-family protein affinity substance pertaining to the present invention, the method for acquisition of small RNA of the present invention is performed as follows. That is, for example, to 1 ml of plasma containing a complex of Ago2 and small RNA, 1 mg to 20 mg, preferably 5 mg to 10 mg of the carrier of the present invention is added, and the mixture is reacted at 2°C to 10°C for 2 h to 24 h. Further, after carrying out centrifugal separation of the obtained reaction solution at 3,000 x g to 5,000 x g for 10 s to 50 s and followed by removing the supernatant solution, the carrier to which the complex has been bound is washed several times using 1 ml to 5 ml of 10 mM to 100 mM Tris buffer solution (pH 7.0 to pH 8.0) containing 0.05 w/v % poly(oxyethylene) nonylphenyl ether and 100 mM to 300 mM NaCl for 1 ml of the reaction solution. Subsequently, for example, by heating at 80°C to 100°C for 90 s to 180 s using citrate buffer solution of pH 4.5 to pH 6.0, objective small RNA is released. After that, the obtained solution is subjected to centrifugal separation at 3,000 x g to 5000 x g for 10 s to 50 s, the supernatant solution is isolated preparatively, and thereby objective small RNA can be obtained.

Since the method for acquisition of a small RNA of the present invention does not require extraction and precipitation procedures of small RNA, by utilizing (incorporating) it as one step of high throughput method, solution processing is possible and small RNA can be obtained simply. Furthermore, the method of the present invention takes such effect that the small RNA can be obtained at equivalent or higher efficiency than by the conventional method.

Hereinafter, the present invention will be explained in more detail by referring to Examples and Comparative Examples.

### Examples

### Example 1: Acquisition of miRNA from human plasma by immunoprecipitation heat elution method (acquisition target: miR 92a)

### (1)Preparation of anti-human Ago2 antibody-immobilized carrier

Anti-human Ago2 antibody-immobilized carrier was prepared as follows using Antibody Immobilization Kit IP (produced by Wako Pure Chemical Industries Ltd.), and 1 mg/ml anti-human Ago2 antibody (produced by Wako Pure Chemical Industries Ltd.)

That is, to 200 mg of Antibody Immobilization Beads, 9.0 ml of Antibody Immobilization Buffer and 1.0 ml of 1 mg/ml anti-human Ago2 antibody were added and mixed, and the mixture was blended by rotating at 37°C for 4 h. After centrifugal separation (3,000 x g for 1 min), the supernatant solution was removed using pipette, and then the beads were washed three times with 10 ml of Washing Buffer (Neutral). After that, 10 ml of Blocking Buffer was added and the mixture was blended by rotating at room temperature for 1 h. After centrifugal separation (3,000 x g for 5 min), the supernatant solution was removed using pipette, and the beads were washed 5 times with 10 ml of Washing Buffer, and once with 10 ml of Storage Buffer, then the beads were suspended in 5.0 ml of Storage Buffer to obtain anti-human Ago2 antibody-immobilized carrier solution (hereinafter, the solution obtained in such a way is described as anti-human Ago2 antibody-immobilized carrier solution).

### (2) Pretreatment of human plasma sample

After centrifugal separation of 1 ml of human pooled plasma (produced by Kohjin Bio Co., Ltd.) (20000 x g for 15 min), supernatant solution was transferred to a fresh tube, and poly(oxyethylene) nonylphenyl ether (produced by Wako Pure Chemical Industries Ltd.) was added thereto so that its final concentration becomes 0.1 w/v %. The solution prepared in such way was used as a human plasma sample (hereinafter, the human plasma sample pretreated in this way is described as pretreated human plasma sample).

### (3) Acquisition of miRNA from human plasma by immunoprecipitation heat elution method

After centrifugal separation of 50 µl of anti-human Ago2 antibody-immobilized carrier solution (corresponding to 2 mg of beads content) (3,000 x g for 30 s), supernatant solution was removed, and the carrier was washed once with 1 ml of 20 mM aqueous Tris hydrochloric acid containing 0.05 w/v % poly(oxyethylene) nonylphenyl ether (produced by Wako Pure Chemical Industries Ltd.) and 200 mM sodium chloride (pH 7.4, hereinafter abbreviated to immunoprecipitation washing liquid), and 200 µl of above-described pretreated human plasma sample was added thereto, and then blended by rotating under refrigeration for 3 h. After centrifugal separation (3,000 x g, for 30 s), the supernatant solution was removed, and the carrier was washed three times with 1 ml of immunoprecipitation washing liquid. After that, 50 µl of nuclease-free water (produced by Nippon Gene Co., Ltd.) was added to the pellet, and reacted at 95°C for 5 min, while the mixture was kept agitated at 1,400 rpm using Thermomixer (produced by Eppendorf GmbH). After centrifugal separation (3,000 x g, for 30 s), the supernatant solution was isolated preparatively, and the solution obtained was used as a sample for measurement.

### (4) Reverse transcription reaction and quantitative PCR of miRNA

Reverse transcription reaction of miRNA was carried out as follows using TaqMan MicroRNA Reverse Transcription Kit (produced by Applied Biosystems Inc.) and TaqMan MicroRNA Assay Kit (for hsa-miR-92a) (produced by Applied Biosystems Inc.).

That is, to 5µL of the sample for measurement obtained by the above-described method, 0.15 µl of 100 mM dNTP mix, 1.5 µl of 10 x RT Buffer, 0.19 µl of 20 U/µl RNase Inhibitor, 1 µl of 50 U/µl Multiscribe RT enzyme, 3 µl of 5 x RT primer (for hsa-miR-92a), and 4.16 µl of purified water were added, and 15 µl in total of the reaction solution was prepared. After carrying out incubation at 16°C for 30 min, and at 42°C for 30 min, each reaction solution was further incubated at 85°C for 5 min to obtain reverse transcription reaction products.

To 1 µl of the obtained reverse transcription reaction product, 7.5 µl of TaqMan 2 x PCR Master Mix (produced by Applied Biosystems Inc.), 0.75 µl of 20 x TaqMan Assay Mix (for hsa-miR-92a), and 5.75 µl of purified water were added, and 15 µl in total of the reaction solution was prepared. The quantitative PCR detection was performed for the reaction solution using ABI7500 Fast Real-Time PCR System (produced by Applied Biosystems Inc.), and respective Ct values were determined.

On the other hand, using a single-stranded synthetic miR-92a (5'-UAUUGCACUUGUCCCGGCCUGU-3') as a standard RNA, dilute solutions by 10 fold dilution to 10¹ copies/µl to 10⁵ copies/µl with purified water were prepared, and the reverse transcription reaction was carried out by the same procedure as described above, and quantitative PCR detection was performed. Standard curve of miR-92a was made from obtained Ct values for each dilute standard RNA solution and its number of molecules. Using the said standard curve, the number of molecules of miR-92a was calculated from each Ct value obtained as described above. The results are shown in Fig. 1.

### Comparative Example 1: Acquisition of miRNA from human plasma by immunoprecipitation SDS-phenol/chloroform elution method (acquisition target: miR 92a)

### (1) Acquisition of miRNA from human plasma by immunoprecipitation SDS-phenol/chloroform elution method

After centrifugal separation (3,000 x g, for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid, then 200 µl of pretreated human plasma sample was added thereto, and the mixture was blended by rotating under refrigeration for 3 h. After centrifugal separation (3,000 x g, for 30 s), the supernatant solution was removed, and the carrier was washed 3 times with 1 ml of the immunoprecipitation washing liquid, to obtain a precipitate (pellet). After that, 50 µl of 0.5 w/v % SDS solution was added to the pellet, and the protein adsorbed on the carrier was eluted. Further, to the eluate solution, 350 µl of sterilized water, and 400 µl of phenol/chloroform/isoamyl alcohol (25:24:1) were added and the solution was mixed by vortex mixer, and then centrifuged (20,000 x g, for 10 min). The upper layer was isolated preparatively, and 400 µL of chloroform was added thereto. The solution was mixed by vortex mixer, and then centrifuged (20,000 x g, for 10 min). The upper layer was isolated preparatively, and 3 µl of Ethachinmate (produced by Nippon Gene Co., Ltd.), 40 µl of 3 M sodium acetate, 1 ml of ethanol were added thereto. The solution was suspended by vortex mixer, and then centrifuged (20,000 x g, for 15 min). Obtained precipitate was washed with 1 ml of 70 v/v % ethanol, and air dried at room temperature for 20 min, and then the dried precipitate was dissolved in 50 µl of nuclease-free water, and this solution was used as a sample for measurement.

### (2) The reverse transcription reaction and quantitative PCR of miRNA

Using the obtained sample for measurement, reverse transcription reaction and quantitative PCR were carried out in the same way as the method described in Example 1 (4), and the number of miR-92a molecules was determined from obtained Ct value. The result was shown in Fig. 1 together with the result of Example 1.

### Comparative Example 2: Acquisition of miRNA from human plasma by immunoprecipitation acidic elution method (acquisition target: miR 92a)

### (1) Acquisition of miRNA from human plasma by immunoprecipitation acidic elution method

After centrifugal separation (3,000 x g, for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid, then 200 µl of pretreated human plasma sample was added thereto, and the mixture was blended by rotating under refrigeration (at about 5°C) for 3 hours. After centrifugal separation (3,000 x g, for 30 s), the supernatant solution was removed, and the carrier was washed 3 times with 1 ml of the immunoprecipitation washing liquid, to obtain a precipitate (pellet). After that, 46.5 µl of 0.1 M glycine-hydrochloric acid buffer solution (pH 2.5) was added to said pellet, and mixed by vortex mixer, and then centrifuged (3,000 x g, for 30 s). The supernatant solution was isolated preparatively and neutralized by addition of 3.5 µl of 1 M CAPS (pH 11.0), and was used as a sample for measurement.

### (2) The reverse transcription reaction and quantitative PCR of miRNA

Using the obtained sample for measurement, reverse transcription reaction and quantitative PCR were carried out by the same method as described in Example 1 (4), and the number of miR-92a molecules was determined from the obtained Ct value. The result is shown in Fig. 1 together with the result of Example 1.

From the results shown in Fig. 1, it is clear that, after plasma Ago2-miRNA complex is immunoprecipitated using anti-Ago2 antibody, by performing the method which is conventionally used as a method for eluting small RNA in the immunoprecipitation method in which phenol/chloroform extraction after SDS elution and ethanol precipitation are carried out, the miRNA bound to Ago2 in plasma can be recovered. However, when acidic 0.1 M glycine hydrochloric acid buffer solution (pH 2.5) was used, the recovery of miRNA was low as compared with the case where phenol/chloroform extraction after SDS elution and ethanol precipitation were carried out.

On the other hand, it turned out that when miRNA elution was carried out by heat treatment of the present invention, even though the eluate solution was applied directly to the reverse transcription reaction, the miRNA could be detected at the same level as detected by the method in which phenol/chloroform extraction after SDS elution and ethanol precipitation were carried out. That is, it was shown that the elution of small RNA such as miRNA by said heat treatment does not require the procedures of nucleic acid extraction by phenol/chloroform and ethanol precipitation, and it was a simple method compatible with high throughput processing.

### Example 2: Effects of different heating temperature on elution efficiency (acquisition target: miR 92a)

### (1) Acquisition of miRNA from human plasma by immunoprecipitation heat elution method

After centrifugal separation (3,000 x g, for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid. Subsequently, 200 µl of pretreated human plasma sample was added thereto, and the mixture was blended by rotating under refrigeration for 3 h. After centrifugal separation (3,000 x g, for 30 s), the supernatant solution was removed, and the carrier was washed 3 times with 1 ml of the immunoprecipitation washing liquid, to obtain a precipitate (pellet). After that, 50 µl of nuclease-free water (produced by Nippon Gene Co., Ltd.) was added to the pellet, and agitated at 1,400 rpm using Thermomixer (produced by Eppendorf GmbH). Six kinds of solutions obtained by the same procedure as described above were prepared, and each of them was reacted independently at 50°C, 60°C, 70°C, 80°C, 90°C, and 100°C for 5 min, while the mixture was kept agitated at 1,400 rpm using Thermomixer. After centrifugal separation (3,000 x g, for 30 s), the supernatant solution was isolated preparatively, and the solution obtained was used as a sample for measurement.

### (2) The reverse transcription reaction and quantitative PCR of miRNA

Using the obtained sample for measurement, reverse transcription reaction and quantitative PCR were carried out by the same method as described in Example 1 (4), and the number of miR-92a molecules was determined from the obtained Ct value. The results were shown in Fig. 2.

From the results shown in Fig. 2, it turned out that the elution efficiency of miRNA from the carrier obtained by immunoprecipitation method showed high elution rate at higher elution temperature, and the miRNA was eluted efficiently at 70°C or higher, and more efficiently at 80°C or higher.

### Example 3: Effects of different heating temperature and heating time on elution efficiency (acquisition target: miR 92a)

### (1) Acquisition of miRNA from human plasma by immunoprecipitation heat elution method

After centrifugal separation (3,000 x g, for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid. Subsequently, 200 µl of pretreated human plasma sample was added thereto, and the mixture was blended by rotating under refrigeration for 3 h. Further, after centrifugal separation (3,000 x g, for 30 s), the supernatant solution was removed, and the carrier was washed 3 times with 1 ml of the immunoprecipitation washing liquid, and obtained a precipitate (pellet). To said pellet, 50 µl of nuclease-free water (produced by Nippon Gene Co., Ltd.) was added. Twenty four kinds of solutions obtained by the same procedure as described above were prepared, and each of them was reacted at 70°C, 80°C, 90°C, and 100°C for 10, 30, 60, 90, 120, and 180 s in combination respectively, while the mixture was kept agitated at 1,400 rpm using Thermomixer (produced by Eppendorf GmbH). Finally, after centrifugal separation (3,000 x g, for 30 s), the supernatant solution was isolated preparatively, and the obtained solution was used as an immunoprecipitation eluate.

### (2) Reverse transcription reaction and quantitative PCR of miRNA

Using obtained sample for measurement, reverse transcription reaction and quantitative PCR were carried out by the same method as described in Example 1 (4), and the number of miR-92a molecules was determined from the obtained Ct value. The results were shown in Fig. 3.

From the results shown in Fig. 3, it is clear that, by the heat treatment at a heating temperature of 70°C for 120 s or longer, at a heating temperature of 80°C for 90 s or longer, or at a heating temperature of 90°C or higher for 30 s or longer, the miRNA can be recovered efficiently from Ago2-miRNA complex bound to anti-human Ago2 antibody-immobilized carrier.

### Example 4: Effects of difference in type and pH of buffer solution for heating on elution efficiency (acquisition target: miR 92a)

### (1) Acquisition of miRNA from human plasma by immunoprecipitation heat elution method

After centrifugal separation (3,000 x g, for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid. Subsequently, 200 µl of pretreated human plasma sample was added thereto, and the mixture was blended by rotating under refrigeration for 3 h. Further, after centrifugal separation (3,000 x g, for 30 seconds), the supernatant solution was removed, and the carrier was washed 3 times with 1 ml of the immunoprecipitation washing liquid, and obtained a precipitate (pellet). Eleven kinds of solutions obtained by the same procedure as described above were prepared, and to each pellet, 50 µl each of nuclease-free water (produced by Nippon Gene Co., Ltd.), 10 mM citrate buffer solution (pH 3.0), 10 mM citrate buffer solution (pH 4.0), 10 mM citrate buffer solution (pH 4.5), 10 mM citrate buffer solution (pH 5.0), 10 mM citrate buffer solution (pH 5.5), 10 mM citrate buffer solution (pH 6.0), 10 mM MES buffer solution (pH 6.5), 10 mM phosphate buffer solution (pH 7.0), 10 mM phosphate buffer solution (pH 8.0) or 10 mM Tris hydrochloride buffer solution (pH 8.0) was added respectively, and reacted at 90°C for 90 s, while the mixture was kept agitated at 1,400 rpm using Thermomixer (produced by Eppendorf GmbH). Finally, after centrifugal separation (3,000 x g, 30 s), the supernatant solution was isolated preparatively, and the solution obtained was used as a sample for measurement.

### (2) Reverse transcription reaction and quantitative PCR of miRNA

Using obtained sample for measurement, reverse transcription reaction and quantitative PCR were carried out by the same method as described in Example 1 (4), and the number of miR-92a molecules was determined from the obtained Ct value. The results were shown in Fig. 4.

From the results shown in Fig. 4, it turned out that as a solution to be used for the heat elution method, along with water, many kinds of buffer solutions, including citrate buffer solution, Good's buffer solution such as MES and phosphate buffer solution could be used. In addition, it also turned out that when the buffer solutions of pH 3.0 to pH 8.0 were used, RNA could be eluted in the same or higher efficiency as eluted using water, and when the buffer solution of pH 4.5 to pH 7.0 was used, RNA could be eluted more efficiently than eluted with water.

### Example 5: Effects of additive agents to be added to the solution for heating on elution efficiency (acquisition target: miR 92a)

### (1) Acquisition of miRNA from human plasma by immunoprecipitation heat elution method

After centrifugal separation (3,000 x g for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid. Subsequently, 200 µl of pretreated human plasma sample was added thereto, and the mixture was blended by rotating under refrigeration for 3 h. Further, after centrifugal separation (3,000 x g for 30 s), the supernatant solution was removed, and the carrier was washed 3 times with 1 ml of the immunoprecipitation washing liquid, and obtained a precipitate (pellet). Seven kinds of solutions obtained by the same procedure as described above were prepared, and to each pellet, 50 µl of nuclease-free water (produced by Nippon Gene Co., Ltd.), nuclease-free water added with E. coli ribosomal RNA (rRNA, produced by Wako Pure Chemical Industries Ltd.) at the final concentration of 0.1 ng/µl, 1 ng/µl, and 10 ng/µl, and nuclease-free water added with salmon sperm DNA (ssRNA, produced by Wako Pure Chemical Industries Ltd.) at the final concentration of 0.1 ng/µl, 1 ng/µl, and 10 ng/µL were added, respectively, and reacted at 90°C for 90 s, while the mixture was kept agitated at 1,400 rpm using Thermomixer (produced by Eppendorf GmbH). After centrifugal separation (3,000 x g, for 30 s), the supernatant solution was isolated preparatively, and the solution obtained was used as a sample for measurement.

### (2) Reverse transcription reaction and quantitative PCR of miRNA

Using obtained sample for measurement, reverse transcription reaction and quantitative PCR were carried out by the same method as described in Example 1 (4), and the number of miR-92a molecules was determined from the obtained Ct value. The results were shown in Fig. 5.

From the results shown in Fig. 5, it is clear that by adding E. coli ribosomal RNA in the range of final concentration between 0.1 ng/µl and 10 ng/µl, or by adding salmon sperm DNA in the range of final concentration between 0.1 ng/µl and 1 ng/µl to the solution to be used for heat elution method, the recovered amount of miRNA is increased. That is, it turned out that by adding an appropriate amount of nucleic acid to the heating solution, the elution of miRNA was increased. This was assumed to be due to the effects of protection of miRNA from heat, and the effects of protection of miRNA from adsorption to the tube as a container.

### Example 6: Immunoprecipitation heat elution method using various kinds of solutions for heat elution (acquisition target: miR 92a)

### (1) Acquisition of miRNA from human plasma by immunoprecipitation heat elution method

After centrifugal separation (3,000 x g, for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid. Subsequently, 200 µl of pretreated human plasma sample was added thereto, and the mixture was blended by rotating under refrigeration for 3 hours. After centrifugal separation (3,000 x g, for 30 s), the supernatant solution was removed, and the carrier was washed 3 times with 1 ml of the immunoprecipitation washing liquid, and obtained a precipitate (pellet). Four kinds of solutions obtained by the same procedure as described above were prepared, and to each pellet, 50 µl of nuclease-free water (produced by Nippon Gene Co., Ltd.), nuclease-free water added with E. coli ribosomal RNA (rRNA, produced by Wako Pure Chemical Industries Ltd.) at the final concentration of 1 ng/µl, nuclease-free water added with salmon sperm DNA (ssRNA, produced by Wako Pure Chemical Industries Ltd.) at the final concentration of 1 ng/µl, and 10 mM citrate buffer solution (pH 5.5) were added respectively, and reacted at 90°C for 90 s, while the mixture was kept agitated at 1,400 rpm using Thermomixer (produced by Eppendorf GmbH). After centrifugal separation (3,000 x g, for 30 s), the supernatant solution was isolated preparatively, and the solution obtained was used as a sample for measurement.

### (2) Reverse transcription reaction and quantitative PCR of miRNA

As for the measurement of the amount of miR-92a, using obtained sample for measurement, reverse transcription reaction and quantitative PCR were carried out by the same method as described in Example 1 (4), and the number of miR-92a molecules was determined from the obtained Ct value.

As for the measurement of the amount of miR-16, firstly, using TaqMan MicroRNA Reverse Transcription Kit (produced by Applied Biosystems Inc.) and TaqMan MicroRNA Assay Kit (for hsa-miR-16) (produced by Applied Biosystems Inc.), the reverse transcription reaction was carried out as follows.

That is, to 5µl of the obtained sample for measurement, 0.15 µl of 100 mM dNTP mix, 1.5 µl of 10 x RT Buffer, 0.19 µl of 20 U/µL RNase Inhibitor, 1 µl of 50 U/µL Multiscribe RT enzyme, 3 µl of 5 x RT primer (for hsa-miR-16), and 4.16 µL of purified water were added, and 15 µl in total of the reaction solution was prepared. After carrying out incubation at 16°C for 30 min, and at 42°C for 30 min, each reaction solution was further incubated at 85°C for 5 min to obtain reverse transcription reaction products.

Subsequently, to 1 µl of each obtained reverse transcription reaction product, 7.5 µl of TaqMan 2 x PCR Master Mix (produced by Applied Biosystems Inc.), 0.75 µl of 20 x TaqMan Assay Mix (for hsa-miR-16), and 5.75 µl of purified water were added, and 15 µl in total of the reaction solution was prepared. The quantitative PCR detection was performed for the reaction solution using ABI7500 Fast Real-Time PCR System (produced by Applied Biosystems Inc.), and respective Ct values were determined.

On the other hand, using a single-stranded synthetic miR-16 (5'-UAGCAGCACGUAAAUAUUGGCG -3') as a standard RNA, dilute solutions by 10 fold dilution to 10¹ copies/µl to 10⁵ copies/µl with purified water were prepared, and for 1µl of each dilute solution, the reverse transcription reaction was carried out by the same procedure as described above, and quantitative PCR detection was performed. Standard curve of miR-16 was prepared from obtained Ct values for each dilute standard RNA solution and its number of molecules. Using the aforementioned standard curve, the number of molecules of miR-16 was calculated from each Ct value obtained as described above. The results are shown together with the results of miR-92a in Fig. 6.

### Comparative Example 3: Acquisition of miRNA from human plasma by immunoprecipitation SDS-phenol/chloroform elution method (acquisition target: miR-16, miR 92a)

### (1) Acquisition of miRNA from human plasma by immunoprecipitation SDS-phenol/chloroform elution method

After centrifugal separation (3,000 x g, for 30 s) of 50 µl of anti-human Ago2 antibody-immobilized carrier solution (corresponding to 2 mg of beads content), the supernatant solution was removed, and the carrier was washed once with 1 ml of immunoprecipitation washing liquid, then 200 µl of pretreated human plasma sample was added thereto, and the mixture was blended by rotating under refrigeration for 3 hours. After centrifugal separation (3,000 x g, for 30 s), the supernatant solution was removed, and the carrier was washed 3 times with 1 ml of the immunoprecipitation washing liquid, and obtained a precipitate (pellet). After that, 50 µl of 2 w/v % SDS solution was added to the pellet, and the protein adsorbed on the carrier was eluted. To the eluate, 350 µl of sterilized water, and 400 µl of phenol/chloroform/isoamyl alcohol (25:24:1) were added and the solution was mixed by vortex mixer, and then centrifuged (20,000 x g, for 10 min). The upper layer was isolated preparatively, and 400 µl of chloroform was added thereto, and the solution was mixed by vortex mixer, and then centrifuged (20,000 x g, for 10 min). The upper layer was isolated preparatively, and 3 µl of Ethachinmate (produced by Nippon Gene Co., Ltd.), 40 µl of 3 M sodium acetate, and 1 ml of ethanol were added thereto. The solution was mixed by vortex mixer, and then centrifuged (20,000 x g, for 15 min).

Obtained precipitate was washed with 1 ml of 70 v/v % ethanol, and air dried at room temperature for 20 min, and then the dried precipitate was dissolved in 50 µl of nuclease-free water, and this solution was used as a sample for measurement.

### (2) Reverse transcription reaction and quantitative PCR of miRNA

Using obtained sample for measurement, reverse transcription reaction and quantitative PCR were carried out in the same way as the method described in Example 6 (2), and the number of miR-16 and miR-92a molecules were determined from the obtained Ct values. The result was shown in Fig. 6 together with the results of Example 6.

### Comparative Example 4: Acquisition of miRNA from human plasma by AGPC method (conventional RNA purification method) (acquisition target: miR-16, miR 92a)

### (1) Purification of miRNA by AGPC method

To 200 µl of pretreated human plasma sample, 600 µl of ISOGEN-LS (produced by Nippon Gene Co., Ltd.) and 2 µl of glycogen (produced by Wako Pure Chemical Industries Ltd.) were added and mixed by vortex mixer for 2 minutes, and then 160 µl of chloroform was added thereto and mixed by vortex mixer for 10 min. Further, after centrifugal separation (20,000 x g, for 10 min), the upper layer was isolated preparatively, 0.6 ml of isopropanol was added thereto and mixed, and then centrifuged (20,000 x g, for 10 min). Subsequently, precipitate was taken out, and after washing with 1 ml of 70 v/v % ethanol, air dried at room temperature for 20 min. Obtained dried precipitate was dissolved in 50 µl of sterilized water, and this solution was used as a sample for measurement.

### (2) Reverse transcription reaction and quantitative PCR of miRNA

Using each obtained sample for measurement, reverse transcription reaction and quantitative PCR were carried out in the same way as the method described in Example 6 (2), and the number of miR-16 and miR-92a molecules was determined. The result was shown in Fig. 6 together with the results of Example 6 and Comparative Example 3.

From the results shown in Fig. 6, it is clear that, in the immunoprecipitation heat elution method, either of nuclease-free water added with E. coli ribosomal RNA (final concentration: 1 ng/ul, nuclease-free water added with salmon sperm DNA (final concentration: 1 ng/µl), or 10 mM citrate buffer solution (pH 5.5) is used for heating treatment, miR-16 in plasma can be obtained in more efficiently than the conventional immunoprecipitation method in which phenol/chloroform extraction after SDS elution and ethanol precipitation are carried out, or AGPC method which is commonly used for RNA purification.

In addition, with respect to miR-92a, according to the immunoprecipitation heat elution method of the present invention, the use of either solution also resulted in the same or higher recovered amount than that obtained by the conventional immunoprecipitation method in which phenol/chloroform extraction after SDS elution and ethanol precipitation are carried out; and the immunoprecipitation heat elution method with the use of nuclease-free water added with E. coli ribosomal RNA (final concentration: 1 ng/µl), nuclease-free water added with salmon sperm DNA (final concentration: 1 ng/µl), or 10 mM citrate buffer solution (pH 5.5) resulted in almost the same recovered amount to AGPC method.

Therefore, from these results, it turned out that, even though practicable in simple procedure, the immunoprecipitation heat elution method of the present invention was an excellent method which could recover small RNA such as miRNA in the plasma at the same or higher efficiency as compared with the conventional method.

## Claims

1. A method for acquisition of small RNA having 5 to 200 nt, said method comprising contacting a carrier, which comprises a substance having affinity to a small RNA-binding Argonaute-family protein immobilized on its surface, with a complex of the Argonaute-family protein and the small RNA (Argonaute-family protein-RNA complex) to bind the Argonaute-family protein-RNA complex to the aforementioned carrier, and
releasing the small RNA by heating the carrier bound with the aforementioned Argonaute-family protein-RNA complex at 70°C to 100°C for 30 to 600 s in water or in a buffer solution of pH 3.0 to pH 8.0.

2. The method according to claim 1, wherein after the Argonaute-family protein-RNA complex is bound to the carrier, and before heating, the obtained carrier bound with the Argonaute-family protein-RNA complex is washed.

3. The method according to claim 1 or 2, wherein the carrier is heated in a buffer solution.

4. The method according to any one of claim 1 to 3, wherein the pH of the buffer solution is 4.5 to 7.0.

5. The method according to any one of claim 1 to 4, wherein the buffer solution is a citrate buffer solution, Good's buffer solution or phosphate buffer solution.

6. The method according to any one of claim 1 to 5, wherein the water or the buffer solution contains RNA or DNA which does not comprise any nucleotide sequence of the small RNA.

## Patentansprüche

1. Verfahren zur Gewinnung kleiner RNA mit 5 bis 200 nt, wobei das Verfahren das In-Kontakt-Bringen eines Trägers, der eine Substanz mit Affinität zu einem Protein aus der kleine RNA bindenden Argonautenfamilie, die auf seiner Oberfläche immobilisiert ist, umfasst, mit einem Komplex aus dem Protein der Argonautenfamilie und der kleinen RNA (Argonautenfamilieprotein-RNA-Komplex), um den Argonautenfamilieprotein-RNA-Komplex an den oben genannten Träger zu binden, und das Freisetzen der kleinen RNA durch Erhitzen des Trägers, an den der oben genannte Argonautenfamilieprotein-RNA-Komplex gebunden ist, auf 70 °C bis 100 °C während 30 bis 600 s in Wasser oder in einer Pufferlösung von pH 3,0 bis pH 8,0 umfasst.

2. Verfahren gemäß Anspruch 1, wobei der erhaltene Träger, an den der Argonautenfamilieprotein-RNA-Komplex gebunden ist, gewaschen wird, nachdem der Argonautenfamilieprotein-RNA-Komplex an den Träger gebunden wurde, und vor dem Erhitzen.

3. Verfahren gemäß Anspruch 1 oder 2, wobei der Träger in einer Pufferlösung erhitzt wird.

4. Verfahren gemäß einem der Ansprüche 1 bis 3, wobei der pH-Wert der Pufferlösung 4,5 bis 7,0 beträgt.

5. Verfahren gemäß einem der Ansprüche 1 bis 4, wobei die Pufferlösung eine Citrat-Pufferlösung, eine Good-Pufferlösung oder eine Phosphatpufferlösung ist.

6. Verfahren gemäß einem der Ansprüche 1 bis 5, wobei das Wasser oder die Pufferlösung RNA oder DNA enthält, die keine Nucleotidsequenz der kleinen RNA umfasst.

## Revendications

1. Procédé pour l'acquisition de petit ARN ayant de 5 à 200 nt, ledit procédé comprenant
la mise en contact d'un support, qui comprend une substance ayant une affinité à l'égard d'une protéine de la famille Argonaute se liant à un petit ARN immobilisée sur sa surface, avec un complexe de la protéine de la famille Argonaute et du petit ARN (complexe protéine de la famille Argonaute-ARN) afin de lier le complexe protéine de la famille Argonaute-ARN au support susmentionné, et
la libération du petit ARN en chauffant le support lié au complexe protéine de la famille Argonaute-ARN susmentionné à une température de 70 °C à 100 °C pendant 30 à 600 s dans de l'eau ou dans une solution tampon de pH 3,0 à pH 8,0.

2. Procédé selon la revendication 1, dans lequel, après la liaison du complexe protéine de la famille Argonaute-ARN au support, et avant le chauffage, on lave le support lié au complexe protéine de la famille Argonaute-ARN obtenu.

3. Procédé selon la revendication 1 ou 2, dans lequel le support est chauffé dans une solution tampon.

4. Procédé selon l'une quelconque des revendications 1 à 3, dans lequel le pH de la solution tampon est de 4,5 à 7,0.

5. Procédé selon l'une quelconque des revendications 1 à 4, dans lequel la solution tampon est une solution tampon citrate, une solution tampon de Good ou une solution tampon phosphate.

6. Procédé selon l'une quelconque des revendications 1 à 5, dans lequel l'eau ou la solution tampon contient de l'ARN ou de l'ADN qui ne comprend pas de séquence nucléotidique du petit ARN.


### SEQUENCE LISTING

<110> WAKO PURE CHEMICAL INDUSTRIES, LTD.
<120> A method for obtaining small RNA
<130> 1833JP
<160> 2
<170> PatentIn version 3.3
<210> 1
   <211> 22
   <212> RNA
   <213> Artificial
<220>
   <223> miR-92a
<400> 1
   UAUUGCACUUGUCCCGGCCUGU
<210> 2
   <211> 4
   <212> RNA
   <213> Artificial
<220>
   <223> miR-16
<400> 2
   UAGCAGCACGUAAAUAUUGGCG